# EUROPÄISCHE PATENTSCHRIFT

(11) **EP 1 334 702 B1**
(45) Veröffentlichungstag und Bekanntmachung des Hinweises auf die Patenterteilung: **07.07.2004**
(21) Anmeldenummer: 02002861.9
(22) Anmeldetag: 08.02.2002
(51) Int. Cl.: A61F 2/08, A61F 2/28

(54) **Ankerelement zum Verankern eines Ligamenttransplantates**
Anchor element to anchor a ligament transplant
Elément d'ancrage pour ancrer un greffon de ligament

(43) Veröffentlichungstag der Anmeldung: 13.08.2003
(73) Patentinhaber: Karl Storz GmbH & Co. KG, 78532 Tuttlingen (DE)
(72) Erfinder: Timmermans, André, 7261 JH Ruurlo (NL); Gebhardt, Ullrich, 02625 Bautzen (DE)
(74) Vertreter: Weller, Wolfgang, Dr.

(56) Entgegenhaltungen:
- EP-A- 0 642 773
- WO-A-01/82989
- WO-A-93/15694
- WO-A-98/37835
- WO-A-99/44544
- US-A- 4 755 184
- US-A- 5 501 706

## Beschreibung

Die Erfindung betrifft ein Ankerelement zum Verankern eines Ligamenttransplantates in einem Kanal in einem Knochen, mit einem Körper, der in einen Zwischenraum zwischen einer Innenwand des Kanals und der Außenseite des darin eingebrachten Ligamenttransplantates bringbar ist und dadurch das Ligamenttransplantat im Kanal verklemmt.

Ein derartiges Ankerelement ist beispielsweise aus der DE-A-198 51 152 bekannt.

Derartige Ankerelemente werden bei Operationen eingesetzt, bei denen ein beschädigtes Ligament durch ein Ligamenttransplantat ersetzt wird.

Ein typischer Fall ist beispielsweise ein Kreuzbandersatz in einem Kniegelenk.

Solche Operationen werden erforderlich, wenn das Kreuzband im Knie gerissen oder anderweitig stark beschädigt ist.

Als Kreuzbandersatz werden dabei bevorzugt körpereigene Materialien verwendet. Dabei haben sich insbesondere die Patellar-Sehne oder die Semitendinosus-Sehne als geeignet erwiesen den benötigten Kreuzbandersatz herzustellen.

Diese Operationstechnik soll beispielshaft bei dem Ersatz eines Kreuzbandes an einem Kniegelenk erläutert werden. Diese Technik kann auch an anderen Gelenken beispielsweise am Schultergelenk oder auch an anderen Körperteilen angesetzt werden, bei denen Knochen über Bänder miteinander verbunden sind.

Bei dem in der eingangs genannten Druckschrift beschriebenen Verfahren, wird die Semitendinosus-Sehne als Kreuzbandersatz verwendet. Diese wird mit Hilfe eines Sehnenschneiders bei dem Patienten entfernt und anschließend in einzelne Segmente oder Abschnitte unterteilt. Manche dieser Segmente werden in Form einer Schlaufe zusammengelegt, wodurch sich doppelsträngige Segmente ergeben. Diese einzelnen Segmente oder auch die Doppelstränge werden anschließend nebeneinander gelegt und an ihren Enden mit Nahtfäden miteinander verbunden. Werden zwei Doppelstränge miteinander verbunden spricht man von einer sogenannten Quadrupeltechnik. Das auf dieser Weise hergestellte Sehnenbündel bildet den Kreuzbandersatz also das Ligamenttransplantat.

Bei einem Kreuzbandersatz wird das Transplantat zwischen dem distalen Ende des Oberschenkelknochens (das Femur) und dem proximalen Ende des Unterschenkelknochens (der Tibia) befestigt. Dazu wird zunächst im proximalen Ende der Tibia eine Durchgangsbohrung und im distalen Ende des Femur eine Sacklochbohrung gebohrt. In diese beiden Bohrungen wird jeweils ein Ende des Kreuzbandersatzes eingesetzt und in den Bohrungen fixiert.

Der Bohrkanal hat einen kreisförmigen Querschnitt. Wird in einen solchen Bohrkanal ein Ligamenttransplantat in Form eines Doppelstranges eingesetzt so weist dessen Querschnitt etwa die Form einer "8" auf. Demzufolge entsteht ein Zwischenraum zwischen der Innenwand des Bohrkanals und der Außenseite des darin eingebrachten Ligamenttransplantates, der im Beispiel eines doppelstragförmigen Kreuzbandersatzes auf jeder Seite eine etwa doppelsichelförmigen Querschnitt aufweist.

Zur Fixierung des Ligamenttransplantates in dem Kanal sind nun Ankerelemente vorgesehen, die in den Zwischenraum zwischen der Innenwand des Bohrkanales und der Außenseite des Ligamenttransplantates eingetrieben werden.

Aus der US-A-4 755 184 ist ein Implantat für Kieferknochen bekannt, das eine Hülle aus einem porösen Maschenwerk aus resorbierbaren Polyglykolsäurepolymer-Material besitzt. In der Hülle ist ein Prothesenfüllmaterial, bestehend aus Hydroxylapatit aufgenommen. Dieses Implantat dient dazu, einen Knochenersatz zu bilden.

Aus der WO 99/44544 A ist ein Ankerelement bekannt, das durch eine entsprechend geometrische Ausgestaltung, insbesondere durch die Schlitzung längs dessen Längsachse, verformbar ist.

Bei der Eingangs genannten DE-A-198 51 152 bestehen die Ankerelemente in Form von zylindrischen Dübeln. Diese zylindrischen Dübel bestehen aus einem kompaktierten somit nicht plastischen Knochenmaterial, und diese weisen eine solche Größe auf, daß sie als Klemmstücke zwischen der Außenseite des Doppelstranges des Sehnentransplantates und der Innenwand des Bohrkanales wirken. Die zylindrischen Dübel stehen insbesondere im Bereich der Einschnürung der "8" des Doppelstranges mit dem Transplantat in Berührung, auf der gegenüberliegenden Seite mit einem gewissen Umfangsbereich an der Innenseite der kreisförmigen Kanalwand. Die Dübel aus komprimierten Knochenmaterial sind nicht plastisch formbar und deren Härte ist höher als die des Sehnenmaterials, so daß die Sehne durch Verformung etwas gequetscht wird.

Nachteilig daran ist, daß dadurch eine Beeinträchtigung der Sehnen folgen kann, was bei Belastungen, insbesondere den erheblichen Belastungen im Kniegelenk, zu Rupturen führen kann.

Bei Inkongruenz zwischen Bohrkanal und Transplantat schwingt das Band im Kanal und führt bei der Schlußrotation des Gelenkes noch eine Drehbewegung im Kanal aus. Das Transplantat heilt nicht in den spongiösen Bohrkanal ein, es wird nur noch an der Aufhängung und durch Narbengewebe gehalten. Die fortbestehende Instabilität führt zu einem hohen intraartikulären Druck mit ständigem Einpressen von Synovialflüssigkeit in die Bohrkanäle, die Instabilitätsschäden erhöhen sich dadurch.

Durch das Hin- und Herschwingen des Sehnenersatzes in dem Bohrkanal weitet dieser sich zur Außenseite hin trichterartig auf, wobei dies durch harte stiftartige Ankerelemente noch gefördert wird, so daß das in der Fachsprache als "Scheibenwischereffekt" genannte Phänomen auftritt.

Es wurden nun unterschiedliche Anstrengungen unternommen diesem Phänomen entgegen zu wirken.

Aus der US-A-6 001 1001 ist bekannt, harte steife Ankerelemente zu schaffen, die einen möglichst großen Bereich des Zwischenraumes zwischen der Innenwand des Bohrkanales und der Außenseite des Ligamenttransplantates formschlüssig ausfüllen.

Dabei besteht das Problem, daß die Größe und die Form des Sehnentransplantates von zahlreichen Faktoren abhängig ist, also beispielsweise von dem Alter und der Statur des Patienten, und von der angewandten Technik, also einer Einstrangtechnik einer Doppelstrangtechnik oder einer Quadrupelstrangtechnik.

Dies macht eine Vielzahl an unterschiedlich großen und an unterschiedlich geformten steifen Ankerelementen notwendig. Auch damit könnte das Problem des "Scheibwenwischereffektes" nicht beseitigt werden, denn die harten und festen passgeformten Ankerelemente würden ja diese Schwingbewegung auf die den Bohrkanal umgebende Knochenwand übertragen und es kann nicht ausgeschlossen werden, daß nach und nach doch ein Spiel oder die unerwünschte trompetenartige Aufweitung der Mündung des Kanales entsteht.

Bei andere Lösungsansätzen wird versucht die Sehne über sogenannte Fixationsschrauben zu verankern bzw. im Bohrkanal zu fixieren.

Aus der US-A-5 961 521 ist die kombinierte Anwendung von Ankerelementen in einer bestimmten Paßform zusammen mit einer Fixationsschraube bekannt.

Diese Lösung hat nicht nur den Nachteil, daß zahlreiche Bauelemente notwendig sind, sondern es kann nicht ausgeschlossen werden, daß durch die Kombination von Ankerelement und Fixierschraube die Sehne beeinträchtigt wird.

Über dieser Operationstechnik steht immer das Erfordernis einer sicheren und physiologischen Verankerung des Transplantates, die für den Patienten möglichst atraumatisch und angenehm erfolgen soll. Ziel ist das rasche Einwachsen des Ligamenttransplantates, das ja ein Organprodukt des Patienten selbst ist, zu erreichen. Interferrenzschrauben zum Fixieren, beispielsweise aus metallischen Materialien, sind Fremdkörper, die bei späteren Revisionen erst aus dem Körper entfernt werden müssen.

Bei harten Ankerelementen besteht die Gefahr, daß die Verankerung eine zu hohe Klemmkraft entwickelt und daher nicht mehr atraumatisch ist.

Es ist daher Aufgabe der vorliegenden Erfindung hier für Abhilfe zu sorgen und ein Ankerelement zu schaffen, das eine sichere und atraumatische Verankerung, die auf Dauer haltbar ist, ermöglicht. Darüber hinaus soll eine Art an Ankerelement für unterschiedliche Operationstechniken und damit bei unterschiedlichen Sehnentransplantatformen eingesetzt werden können.

Erfindungsgemäß wird die Aufgabe durch das Ankerelement gemäß Anspruch 1 gelöst.

Aufgrund der plastischen Verformbarkeit kann mit einem einzigen Ankerelement flexibel an die jeweilig gegebenen Geometrien des Zwischenraumes angepaßt werden.

Wird also beispielsweise eine einstrangige Sehne mit etwa rechteckförmigem Querschnitt, beispielsweise die Patellar-Sehne in einem Bohrkanal mit rundem Querschnitt eingebracht, paßt sich der plastisch verformbare Körper an die entsprechende Geometrie des Zwischenraums an. Dasselbe Ankerelement kann aber auch in einen Zwischenraum eingesetzt werden, der zwischen einem runden Bohrkanal und einem doppelsträngigen Transplantat auf einer Seite entsteht. Entsprechendes ist auch bei einem Strang, der in der Quadrupeltechnik hergestellt worden ist, möglich. Es wurde festgestellt, daß durch die Anpassung des Körpers an den Zwischenraum eine Lockerung nicht stattfindet, da der Körper im Paßsitz großflächig sowohl mit der Innenwand des Bohrkanales als auch mit der Außenseite des Sehnenimplantates in Berührung steht. Die Traumatisierungsgefahr von Knorpel und Knochen sind im Gegensatz zu schwierig einzubringenden Interferrenzschrauben weitaus geringer. Der sogenannte "Scheibenwischereffekt" wurde ebenfalls nicht mehr beobachtet. Dies kann so erklärt werden, daß durch die vollständige Ausfüllung des Bohrkanalquerschnittes durch das Sehnenmaterial einerseits und den plastisch verformbaren Körper des Ankerelements andererseits eine kompakte, örtlich fixierte, Verbundmasse entstanden ist, die für eine atraumatische Verankerung sorgt, dennoch gewisse Bewegungen bei der Beanspruchung des Sehnentransplantates zuläßt, ohne zu Lockerungserscheinungen zu führen.

Gemäß der Erfindung weist der Körper eine flexible Hülle auf, in der ein plastisch verformbares, teiliges Knochenmaterial aufgenommen ist.

Diese Maßnahme hat den Vorteil, daß durch die flexible Hülle die plastische Verformung entsprechend der Erfindung möglich ist, das Material aufgrund der Hülle aber innerhalb dieser Hülle gehalten wird, so daß ganz definierte Halte- bzw. Verankerungsstellen in einem gewissen abgegrenzten Bereich geschaffen werden, beispielsweise nicht über eine gesamte Länge eines Bohrkanales sondern nur über einen bestimmten Bereich, beispielsweise im Endbereich eines Bohrkanales.

Das teilige Knochenmaterial hat den erheblichen Vorteil, daß das Material selbst sehr hart sein kann und die plastische Verformbarkeit dadurch entsteht, daß aufgrund der Teiligkeit des Materials in der Hülle die gewünschten Formen entstehen können. Man kann sich das wie einen gefüllten Sandsack vorstellen, der entsprechend verformt werden kann, plötzlichen Druckbeanspruchungen, wie beispielsweise beim Bewegen einer Sehne, jedoch gewachsen ist.

In einer weiteren Ausgestaltung der Erfindung weist die Hülle eine löchrige Struktur auf.

Diese Maßnahme hat den Vorteil, daß über die löchrige Struktur Knochenmaterial in den Körper einwachsen kann, so daß dann ein fester Verbund zwischen dem Körper und dem diesem umgebenden Knochen entsteht.

In einer weiteren Ausgestaltung der Erfindung ist das Material der Hülle biodegradierbar.

Diese Maßnahme hat den Vorteil, daß die Hülle nach und nach biologisch abgebaut wird und das in der Hülle vorhandene Material dreidimensional durch eine Knochenmatrix durchwachsen wird.

Gemäß der Erfindung ist das plastisch verformbare Material als teiliges Knochenmaterial ausgebildet.

Dies Maßnahme hat den erheblichen Vorteil, daß eine besonders günstige Integration des Verankerungskörpers stattfinden kann. Ist das teilige Knochenmaterial Material des Patienten selbst, stehen schon körpereigenes Knochenmaterialien bereit, in die dann problemlos körpereigenes Material einwachsen und mit diesem teiligen Knochenmaterial verwachsen kann.

Dazu kann dasjenige Knochenmaterial herangezogen werden, das vorher im Bereich des Bohrkanales entfernt wurde, beispielsweise durch feinteilige Zerteilung dieses Knochenmaterials.

Dazu kann die Hülle zunächst als offene Hülle ausgebildet sein, in die das Knochenmaterial eingefüllt werden kann und die anschließend verschlossen wird, beispielsweise durch Vernähen. Die Hülle ist also quasi als Köcher ausgebildet in die das feinteilige Knochenmaterial des Patienten eingefüllt wird. Die löchrige Struktur der Hülle hat in diesem Zusammenhang den erheblichen Vorteil daß aus der Umgebung durch das Netz hindurch "Einbluten" kann und somit besonders rasch eine Integration des "Fremdkörpers" stattfinden kann.

Gemäß der Erfindung ist der Körper mit einem Zugfaden verbunden.

Diese Maßnahme hat den erheblichen Vorteil, daß die Handhabung, das Einbringen und das Positionieren des Körpers erleichtert ist.

In einer weiteren Ausgestaltung der Erfindung weist der Körper originär eine etwa zylindrische Form auf.

Diese Maßnahme hat den erheblichen Vorteil, daß diese Form ein Einbringen bzw. ein Einziehen in den Zwischenraum erleichtert, wonach durch entsprechende Handhabungen der Körper plastisch in die jeweils vorhandene Form des Zwischenraums geformt wird.

In einer weiteren Ausgestaltung der Erfindung weist der Körper originär eine etwa kegelstumpfförmige Form auf.

Diese Maßnahme hat den Vorteil, daß über das dünnere Ende ein einfacher Ansatz des Ankerelements am Zwischenraum geschaffen ist.

Der Körper kann dabei entweder vorfabriziert sein, je nach Ausgestaltung und Materialien, oder er kann kurz vor der Operation angefertigt werden. Dazu ist beispielsweise die Hülle als Köcher oder lediglich als rechteckformicen Gebilde ausgestaltet, auf das das Knochenmaterial in teiliger Form, beispielsweise als kleine Würfel aufgebracht wird und anschließend dieses Netzwerk zu einem geschlossenen Körper verschlossen, beispielsweise vernäht wird.

Die Erfindung wird nachfolgend anhand einiger ausgewählter Ausführungsbeispiele in Zusammenhang mit den beiliegenden Zeichnungen näher beschrieben und erläutert.

Es zeigen:
- Fig.1: eine Seitenansicht eines erfindungsgemäßen Ankerelementes in Form eines vernähten Köchers aus einer Netzwerkstruktur, der mit einem Zugfaden verbunden ist und der mit kleinen Würfeln aus Spangiosa gefüllt ist,
- Fig.2: einen Querschnitt durch das Ankerelement von Fig.1 längs der Linie II-II,
- Fig.3: stark schematisiert einen Längsschnitt durch einen Oberschenkelknochen in dem gerade ein Ligamenttransplantat eingesetzt wird unter Zuhilfenahme von erfindungsgemäßen Ankerelementen,
- Fig.4: einen stark vergrößerten Querschnitt längs der Linie IV-IV in Fig.3 nach Verankern des Ligamenttransplantates, und
- Fig.5: einen dem Schnitt von Fig.4 vergleichbarer Darstellung bei der Verwendung eines anders geformten Ligamenttransplantates

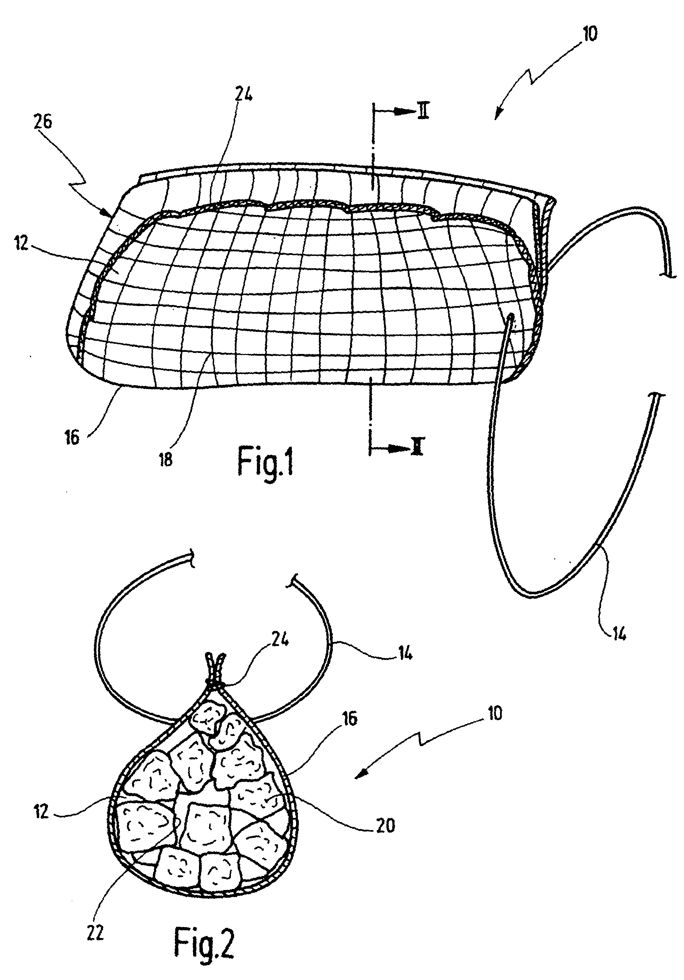

Ein in den Fig. 1 und 2 dargestelltes erfindungsgemäßes Ankerelement ist in seiner Gesamtheit der Bezugsziffer 10 versehen.

Das Ankerelement 10 weist einen grob stabförmigen oder zylindrischen Körper 12 auf, der mit einem Zugfaden 14 verbunden ist.

Die Außenseite des Körpers 12 ist durch eine Hülle 16 abgeschlossen, die eine netzartige Struktur 18 aufweist. Netzartig bedeutet, daß zahlreiche Öffnungen in der Hülle 16 vorhanden sind, die beispielsweise das Eindringen einer Flüssigkeit von der Außenseite in den Innenraum der Hülle 16 ermöglichen. In der Hülle 16 ist ein Material 20 in Form von zahlreichen Würfeln 22 aus Spongiosa aufgenommen. Die Würfel müssen nicht die exakte Form eines Würfels innehaben und weisen eine Größe von etwa 0,5-3 mm auf.

Die Hülle 16 hat in abgewickeltem Zustand eine etwa rechteckige Form und es wurden etwa mittig auf diese Form eine Menge der Würfel 22 aufgelegt. Anschließend wurden zwei gegenüberliegende Seiten umgelegt und aneinandergeklappt und der Körper über eine etwa U-förmige Naht 24 zu einem geschlossenen Körper 12 bzw. Köcher vernäht. Demzufolge umschließt die Hülle 16 die darin aufgenommene plastisch verformbare Masse an Würfeln 22 aus Spongiosa. Die Hülle 16 ist ferner mit einem Zugfaden 14 verbunden, der einfach mit einer Nadel durch den Körper 12 hindurchgestochen wurde. Je nach Ausgestaltung ist der Zugfaden 14 an seinen äußeren Enden miteinander verknotet, somit zu einer Schleife verbunden.

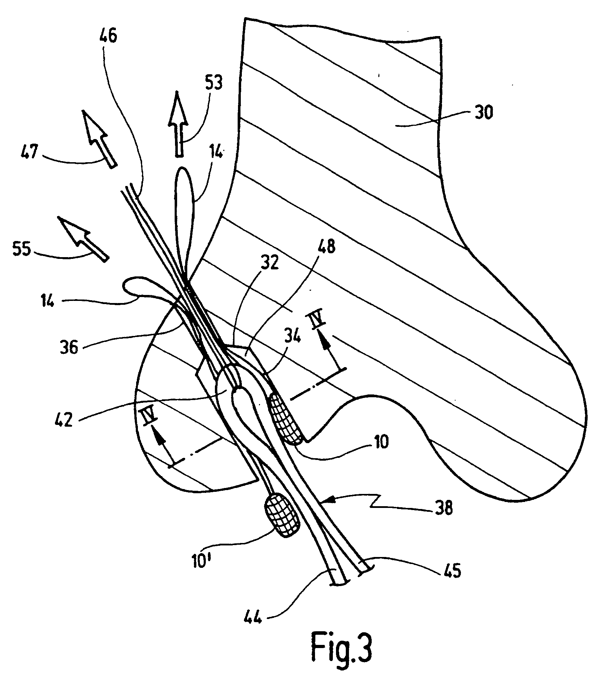

Der Einsatz der erfindungsgemäßen Ankerelemente soll nachfolgend beispielhaft anhand der Fig. 3 bis 5 näher beschrieben werden, wobei es sich hier beispielhaft um einen Kreuzbandersatz in einem Kniegelenk handelt.

In Fig. 3 ist lediglich der Oberschenkel des Kniegelenkes dargestellt. Das dort angedeutete Ligamenttransplantat 38 in Form eines Kreuzbandersatzes erstreckt sich auch bis zum Unterschenkelknochen, wie das an sich bei diese Operationstechnik bekannt ist.

In den Oberschenkelknochen ist, von Seiten des Gelenkes her, nach außen geneigt ein durchgehender Kanal 32 eingebohrt, der einen ersten durchmessergrößeren Abschnitt 34 aufweist, der von einem zweiten durchmessergeringeren Abschnitt 36 gefolgt ist.

Der zweite Abschnitt 36 mündet an der Außenseite des Oberschenkelknochens, der erste Abschnitt 34 im Bereich des Gelenkes. Das eingesetzte Ligamenttransplantat 38 besteht aus einer Semitendinosus-Sehne, die so gelegt wurde, daß zwei nebeneinander verlaufende Stränge 44 und 45 entstehen, die über eine Schleife 42 miteinander verbunden sind.

Im Bereich der Schleife 42 wird dieser Kreuzbandersatz in den durchmessergrößeren ersten Abschnitt 34 des Kanales 32 eingezogen. Dazu ist die Schleife 42 mit mehreren Fäden 46 verbunden, die zuvor durch den Kanal 32 hindurchgefädelt wurden.

Der Operateur kann, wie dies in Fig. 3 durch einen Pfeil 47 dargestellt ist, an den entsprechenden Fäden 46 ziehen und dabei die Schleife 42 in den Kanal einziehen und positionieren.

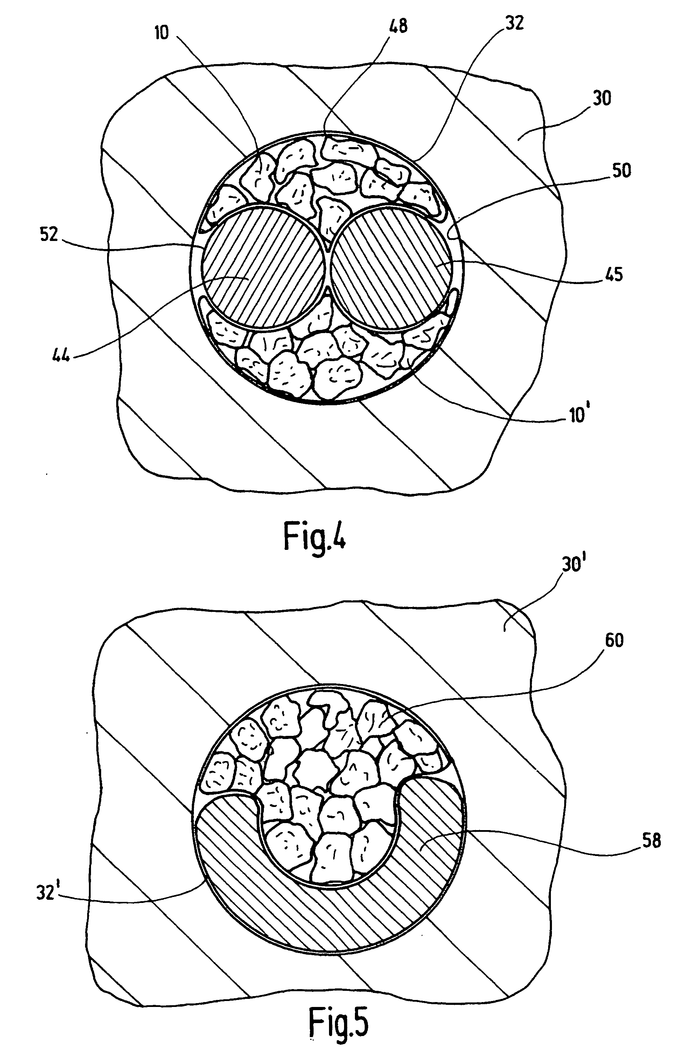

Gleichermaßen ist bereits ein erstes Ankerelement 10 in den ersten durchmessergrößeren Abschnitt 34 eingezogen, wobei dessen Zugfaden 14 ebenfalls vorher durch den Kanal 32 hindurchgefädelt wurde und über die Mündung des zweiten durchmessergeringeren Abschnittes 36 hinausragt. Durch Ziehen an dem Zugfaden 14, wie dies in Fig. 3 durch einem Pfeil 53 angedeutet ist, kann der Körper des Ankerelementes 10 in den Zwischenraum 48 zwischen der Innenwand 50 des durchmessergrößeren Abschnittes 34 und der Außenseite 52 des Ligamanttransplantates 38 verbracht werden, wie das in Fig. 4 an dem oberen Ende ersichtlich ist. Aufgrund der plastischen Verformbarkeit nimmt der Körper 12 des Ankerelementes 10 die Form dieses Zwischenraumes 48 ein, also etwa die Form einer Doppelsichel.

In Fig. 3 ist das Ligamenttransplantat 38 etwas verdreht angedeutet, um die Doppelsträngigkeit zu zeigen. Nach Einziehen des Ligamenttransplantates 38 kann nun auch noch das zweite Ankerelement 10' eingezogen werden, in dem an dessen ebenfalls vorher durch den Kanal 32 durchgefädelten Zugfaden 14' gezogen wird, wie das in Fig. 3 durch den Pfeil 55 angedeutet ist.

Wie insbesondere aus der Schnittdarstellung von Fig. 4 zu erkennen, füllen die Ankerelemente 10, 10' beidseits des doppelsträngigen Ligamenttransplantates 38 vorhandenen Zwischenraum 48 nahezu vollständig aus, so daß ein exakter verankerter Sitz geschaffen ist.

Hier wurde eine Operationstechnik beschrieben, bei der beidseits des doppelsträngigen Ligamenttransplantates 38 jeweils ein Ankerelement 10 bzw. 10' eingesetzt worden ist.

Es ist auch denkbar, den Doppelstrang an eine Seite anzulegen und nur ein Ankerelement zum Verankern einzubringen.

Insbesondere aus der Schnittdarstellung aus Fig. 4 ist zu erkennen, daß bei einem Hin- und Herbewegen des Transplantates 38, beispielsweise in der Darstellung von Fig. 4 nach oben oder nach unten, keine trompetenartige Aufweitung des Kanales 32 erfolgt, da durch die plastische Masse der Würfel aus Spongiosa solche Kräfte abgefedert werden können.

Dadurch, daß die Hülle 16 eine löchrige Struktur 18 aufweist, ist ein Eintreten von entsprechenden Flüssigkeiten die das Wachstum von entsprechenden Osteoblasten fördern möglich, so daß alsbald eine innige Verwachsung des Transplantates 38 mit dem Knochen 30 im Bereich der Ankerelemente 10 und 10' erfolgen kann.

Der gesamte Innenraum des Kanales ist durch körpereigene Stoffe des Patienten aufgefüllt, nämlich durch seine körpereigene Semitendinous-Sehne und körpereigene Spongiosawürfel, so daß ein besonders günstiges Einwachsen ohne Abwehrreaktionen möglich ist. Bei der Verwendung eines bioresorbierbaren Materials der Hülle 16, beispielsweise ein biologisch abbaubares VICRYL-Netz, wird dieses alsbald biologisch abgebaut, so daß nur noch körpereigene Materialien vorhanden sind.

Die Zugfäden 14, 14' der Ankerelemente 10 und 10' erleichtern nicht nur die Handhabung beim Einbringen bzw. Einziehen der Ankerelemente 10, 10' sondern sorgen für eine Fixierung zumindest solange, bis das jeweilige Ankerelement 10, 10' eingewachsen ist.

Bei dem in Fig. 5 dargestellten Ausführungsbeispiel ist gleichermaßen in einen Knochen 30' ein entsprechender kreisförmiger Kanal 32' eingebohrt worden und es wurde ein Ligamenttransplantat 58 eingesetzt, das ursprünglich einen etwas langerstreckten rechteckigen Querschnitt aufweist, beispielsweise eine körpereigene Patellar-Sehne. Das entsprechende Ankerelement 60 ist wie die den Ankerelemente 10, 10' ausgebildet und kann aufgrund dessen plastische Verformbarkeit nun den entsprechenden Zwischenraum zwischen dem Kanal 32' und dem Ligamentimplantat 58 ausfüllen, wie das aus der Schnittdarstellung ersichtlich ist.

Da nach dem Einwachsen aller körpereigener Materialien und ggf. einer Biodegratation der entsprechenden Hülle, die Operationsstelle von der Außenseite her, beispielsweise durch Röntgenuntersuchungen, nicht mehr ersichtlich ist, können zu Kontrollzwecken entsprechende Marker in die Ankerelemente eingebracht werden, sei es im Netzwerk sei es zwischen den Würfeln aus spongiösem Material, um dennoch die eigentliche Operationsstelle röntgentechnisch erfassen zu können. Die zuvor beschriebenen Operationstechniken wurden im Zusammenhang mit einem Kreuzbandersatz beschrieben, können aber gleichermaßen auch bei anderen Operationstechniken anderer Gelenke, sei es im Schultergelenk, im Fußgelenk oder anderen Gelenken eingesetzt werden.

## Patentansprüche

1. Ankerelement zum Verankern eines Ligamenttransplantates (38, 58) in einem Kanal (32, 32') in einem Knochen (30, 30'), mit einem Körper (12), der in einem Zwischenraum (48) zwischen einer Innenwand (50) des Kanales (32, 32') und der Außenseite (52) des darin eingebrachten Ligamenttransplantates (38) bringbar ist und dadurch das Ligamenttransplantat (38, 58) im Kanal (32, 32') verklemmt, wobei der Körper (12) derart plastisch verformbar ist, daß er bei Einbringen in den Zwischenraum (48) der jeweiligen Form des Zwischenraumes (48) anpassbar ist, wobei der Körper (12) eine flexible Hülle (16) aufweist, in der ein plastisch verformbares Material (20) aufgenommen ist, und wobei das plastisch verformbare Material (20) teiliges Knochenmaterial (20) ist, und der Körper (12) mit einem Zugfaden (14, 14') verbunden ist.

2. Ankerelement nach Anspruch 1, wobei die Hülle (16) eine löchrige Struktur (18) aufweist.

3. Ankerelement nach Anspruch 1 oder 2, wobei das Material der Hülle (16) biodegradierbar ist.

4. Ankerelement nach einem der Ansprüche 1 bis 3, wobei der Körper (12) originär eine etwa zylindrische Form (26) aufweist.

5. Ankerelement nach einem der Ansprüche 1 bis 3, wobei der Körper originär eine etwa kegelstumpfförmige Form aufweist.

## Claims

1. Anchoring element for anchoring a ligament transplant (38, 58) in a channel (32, 32') in a bone (30, 30'), with a body (12) which can be brought into an intermediate space (48) between an inner wall (50) of the channel (32, 32') and the outer side (52) of the ligament transplant (38) introduced therein, and, as a result, clamps the ligament transplant (38, 58) in the channel (32, 32'), wherein the body (12) is plastically deformable in such a way, that, when it is introduced into the intermediate space (48), it is adaptable to the respective shape of the intermediate space (48), wherein said body (12) has a flexible sheath (16), in which a plastically deformable material (20) is accommodated, and wherein said plastically deformable material (20) is divided bone material (20), and in that the body (12) is joined to a pulling thread (14, 14').

2. Anchoring element of claim 1, wherein the sheath (16) has a perforated structure (18).

3. Anchoring element of claim 1 or 2, wherein the material of the sheath (16) is biodegradable.

4. Anchoring element of anyone of claims 1 through 3, wherein the body (12) originally has an approximately cylindrical shape (26).

5. Anchoring element of anyone of claims 1 through 3, wherein the body originally has an approximately frustoconical shape.

## Revendications

1. Elément d'ancrage pour ancrer une greffe ligamentaire (38, 58) dans le canal (32, 32') d'un os (30, 30'), comprenant un corps (12) susceptible d'être placé dans un espace intermédiaire (48) entre une paroi intérieure (50) du canal (32, 32') et la face extérieure (52) de la greffe ligamentaire (38) qui y est déposée, coinçant ainsi la greffe ligamentaire (38, 58) dans le canal (32, 32'), le corps (12) étant plastiquement déformable de telle sorte que, lorsqu'il est placé dans l'espace intermédiaire (48), il puisse s'adapter à la forme respective de l'espace intermédiaire (48), le corps (12) présentant un manchon flexible (16) dans lequel est logé un matériau (20) plastiquement déformable, et le matériau (20) plastiquement déformable étant de la matière osseuse (20) en morceaux, et le corps (12) étant relié par un fil à traction (14, 14').

2. Elément d'ancrage selon la revendication 1, dans lequel le manchon (16) présente une structure à trous (18).

3. Element d'ancrage selon la revendication 1 ou 2, dans lequel le matériau qui compose le manchon (16) est biodégradable.

4. Element d'ancrage selon l'une des revendications 1 à 3, dans lequel le corps (12) présente, à l'origine, une forme approximativement cylindrique.

5. Elément d'ancrage selon l'une des revendications 1 à 3, dans lequel le corps présente, à l'origine, une forme approximativement en tronc de cône.
